# EUROPEAN PATENT APPLICATION

(11) **EP 2 441 415 A1**
(43) Date of publication of application: **18.04.2012**
(21) Application number: 10187828.8
(22) Date of filing: 16.10.2010
(51) Int. Cl.: A61F 2/18

(54) **Ossicular replacement prosthesis**

(71) Applicant: Toprak, Emin, 35620 Izmir (TR)
(72) Inventor: Toprak, Emin, 35620 Izmir (TR)

(57) **Abstract**

An ossicular replacement prosthesis (60) for implantation in the middle ear of a human is provided. The prosthesis (60) comprises a connecting element (62). The connecting element (62) is configured to be able turn forth and back on a fulcrum in implanted position according to the vibration applied to it. The turning forth and back causes the connecting element (62) to vibrate a structure that the connecting element is in physical contact with. By this way, the invention uses the principle of a lever pivoting about a fulcrum to transfer vibrations from one point to another. In some embodiments, the ossicular prosthesis (60) further includes a linking element (64). The linking element (64) connects the connecting element (62) to the stapes head or footplate.

## Description

### Technical Field

This invention relates to prostheses for the middle ear for replacement, in whole or in part, of one or more ossicles.

### Background Art

Acoustic sound waves within the outer ear lead to mechanical vibrations of the tympanic membrane. These vibrations are transmitted through the ossicular bones to the footplate of the stapes where motions of this structure result in displacements of the fluid of the inner ear. These displacements lead to vibrations of the hair cells located in the cochlea, and then they are translated into nerve impulses. The nerve impulses are sent to the brain via the cochlear nerve and are interpreted in the brain as sound.

The three ossicular bones are the malleus, which is attached to the tympanic membrane, the stapes, which is connected via its footplate to the inner ear, and the incus, which is situated between the malleus and the stapes, and connected to these in an articulated manner. The stapes have a head, two legs and a footplate. The head and two legs of the stapes form the superstructure of the stapes.

Middle ear prostheses are used to improve the sound transmission in various pathological conditions. They are used to transmit the sound from the tympanic membrane to the inner ear in cases when the auditory ossicles of the human middle ear are completely or partially absent or damaged. Two types of middle ear prostheses that are used frequently are partial ossicular replacement prostheses (PORP) and total ossicular replacement prostheses (TORP).

Partial ossicular replacement prostheses connect the tympanic membrane and/or malleus to the head of the stapes when part or whole of the incus is absent. Total ossicular replacement prostheses connect the tympanic membrane and/or malleus to the footplate of the stapes when superstructure of the stapes is absent.

Partial or total ossicular replacement prostheses that connect only the tympanic membrane to the head or footplate of the stapes are prone to tympanic membrane perforation and prosthesis extrusion because they touch the tympanic membrane. These prostheses are also unable to collect the tympanic membrane vibrations efficiently since they touch only limited portion of the tympanic membrane, not the malleus, which collects the vibrations of whole tympanic membrane.

Moreover, with this type of ossicular prostheses, it is very difficult for an ossicular prosthesis to have high stability, which is necessary for optimum sound transmission, in implanted position for the reason that the ossicular prostheses attach the tympanic membrane, which itself is very mobile structure and changes its position according to the changes in ambient air pressure. Thus, the dislocation of the ossicular prosthesis after implantation is not uncommon and leads to prosthesis malfunction in this type of ossicular prostheses.

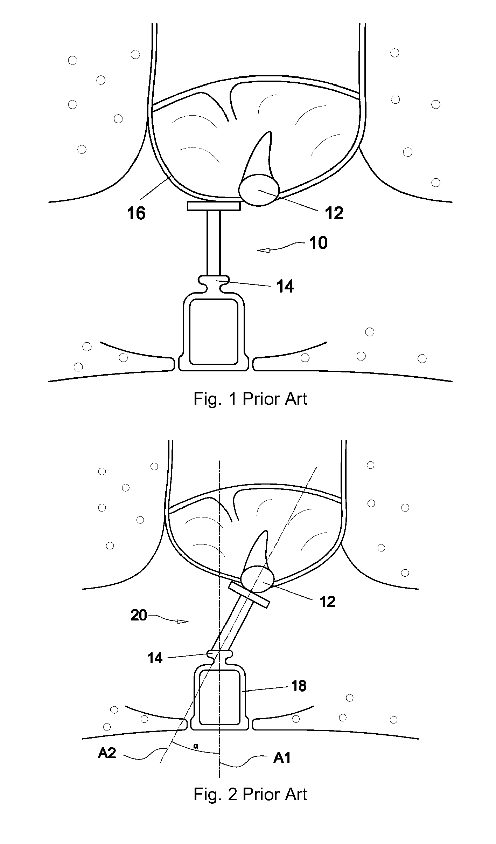

In prior art FIG. 1, an ossicular prosthesis 10 represents this type of ossicular prostheses; the ossicular prosthesis 10 connects only the tympanic membrane 16 to the stapes head 14 and is not in contact with the malleus 12. Ossicular prostheses of this type are known from U.S. Pat. No. 6387128, U.S. Pat. No. 7250059, U.S. Pat. No. 6432139, and U.S. Pat. No. 4676796.

Some partial or total ossicular replacement prostheses may be implanted to connect only the malleus to the stapes head or footplate and avoid direct contact with the tympanic membrane. These types of ossicular replacement prostheses have obvious advantages. It is efficient way of collecting vibration to attach ossicular prostheses directly to the malleus since the malleus collects vibrations from whole tympanic membrane. Furthermore, this type of ossicular prostheses has lower extrusion rates and lower tympanic membrane perforation risk because they do not touch the tympanic membrane.

However, the resulting connection between malleus and stapes head or footplate will be a very oblique connection leading to ineffectual movements of stapes footplate and decreased hearing because the malleus is located in front of the stapes head or footplate. It is well known and documented in the literature that the hearing intensity is directly proportional to amount of displacement of inner ear fluid and that maximum amount of displacement of inner ear fluid is achieved when the vibration of ossicular prosthesis is perpendicular to the stapes head or footplate; in other words, maximum amount of displacement of inner ear fluid is achieved when the ossicular prosthesis vibrates the stapes head or footplate in the vibrational axis of the stapes.

Thus, if the ossicular prosthesis is not perpendicular to the stapes head or stapes footplate, it will cause ineffectual wobbling movements at the footplate, and that diminishes hearing intensity (KB Hüttenbring: Biomechanical Aspects of Middle Ear Reconstruction. In: Jahnke K (ed.): Middle Ear Surgery-Recent Advances and Future Directions, New York, George Thieme Verlag, 2004, p.39). This type of ossicular prostheses is also prone to dislocation because the malleus is located in front of the stapes and the resulting connection between the malleus and the stapes head or footplate will be a very oblique connection, which makes the prosthesis unstable (KB Hüttenbring: Biomechanical Aspects of Middle Ear Reconstruction. In: Jahnke K (ed.): Middle Ear Surgery-Recent Advances and Future Directions, New York, George Thieme Verlag, 2004, p.41).

In prior art FIG. 2, an ossicular prosthesis 20 represents this type of implantations; the ossicular prosthesis 20 connects the malleus 12 to the head 14 of the stapes 18. The vibrational axis of the stapes is represented by axis A1. The ossicular prosthesis has an axis A2. Angle α corresponds to the angle between axis A1 and A2. For optimum vibration transmission, the angle α must be near or equal to zero. This is impossible with this type of ossicular prostheses because of the anatomical relationship of the malleus and the stapes.

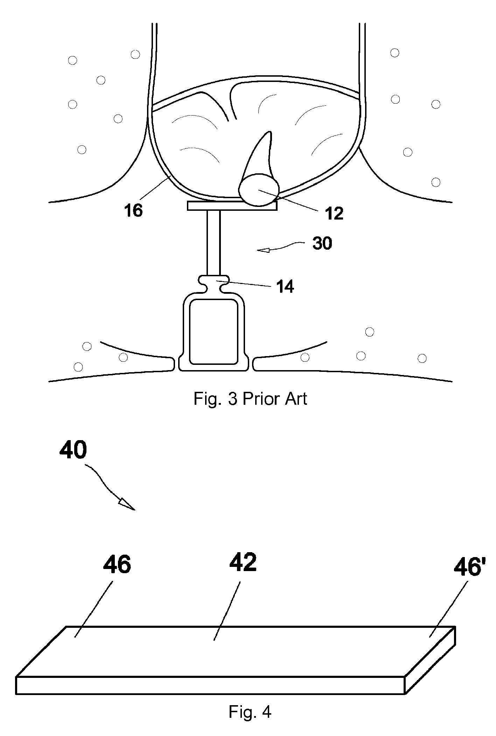

Some partial or total ossicular replacement prostheses are designed to connect the malleus to the stapes head or footplate in such a way that longitudinal axis of the ossicular prosthesis is parallel to the vibrational axis of the stapes; in other words, the ossicular prosthesis is perpendicular to the stapes head or footplate. However, with this type of designs, the ossicular prosthesis frequently does come in undesirable contact with the tympanic membrane, as shown in prior art FIG.3, predisposing the tympanic membrane to erosion, perforation, and eventual prosthesis extrusion. Moreover, vibrations of the malleus are affected by the vibrations in the entire tympanic membrane; therefore, phase of the vibrations in the malleus is not the same as the phase of the vibrations in some part of the tympanic membrane, and so when an ossicular prosthesis connect the both tympanic membrane and the malleus to the stapes head or footplate, vibrations coming to the prosthesis from the tympanic membrane and the malleus cancel out each other due to phase difference, and that leads to ineffective prosthesis. In prior art FIG. 3, an ossicular prosthesis 30 represents this type of prostheses. The ossicular prosthesis 30 connects both the tympanic membrane16 and the malleus 12 to the stapes head 14.

In the view of prior art described above, there is a need for a new ossicular prosthesis that does not have the disadvantages of prior art.

### Summary of invention

The present invention was made in view of the prior art described above.

One aim of the present invention is to provide an ossicular replacement prosthesis that connects only the malleus to the head or footplate of the stapes and applies vibration to the stapes head or footplate perpendicularly; in other words, ossicular replacement prosthesis vibrates the stapes or its footplate in the vibrational axis of the stapes for optimum hearing. Thus, the ossicular replacement prosthesis does not cause tympanic membrane perforation and prosthesis extrusion and does not have the disadvantages of the prior art ossicular prostheses that connects only the malleus to the head or footplate of the stapes.

Another aim of invention is to provide an ossicular replacement prosthesis with improved stability. An optional aim of the invention is to provide an ossicular replacement prosthesis whose length is adjustable so that it is not needed to stock prosthesis with different sizes before operation.

In accord with the invention, an ossicular prosthesis includes a connecting element. The connecting element is configured to be able to turn fort and back on a fulcrum in implanted position when a vibration is applied to the connecting element. This turning movement causes the connecting element to vibrate a structure that the connecting element is in physical contact with. By this way, the invention uses the principle of a lever pivoting about a fulcrum to transfer vibrations.

The embodiments of the invention may further include a linking element. The linking element connects the connecting element to the stapes head or footplate.

The whole or part of the ossicular prosthesis of the invention can be manufactured from titanium, gold, tantalum or an alloy of these metals or any other biocompatible material. For optimum function, however, it is preferable that the ossicular prosthesis is made up of a material that does not fix the structure it touches. Only then the ossicular prosthesis can use the principle of a lever pivoting about a fulcrum efficiently.

Optionally, the part or whole of the prosthesis is manufactured from a malleable material so that prosthesis can be adjusted to different anatomical variations by simply bending it. The whole or parts of the ossicular prosthesis of the invention can be made from a material with shape memory, in particular Nitinol.

According to preferred embodiments of the invention adapted for use where the malleus and the stapes are present, but the incus is partially or completely absent, the ossicular prosthesis includes the connecting element. The connecting element is preferably configured to touch the malleus at one end, a fulcrum at the other end, and the stapes head at somewhere between and spaced apart from ends of the connecting element.

Advantageous refinement of these embodiments provides that the connecting element have one or more notches on between its two ends for better contact with the stapes head.

Another refinement of these embodiments provides that the ossicular prosthesis further includes a linking element. The linking element connects the connecting element to the stapes head or footplate. The linking element may be embodied integrally with the connecting element so that the prosthesis of the invention can be produced in compact and therefore economical form. However, the linking element may be produced separately.

According to preferred embodiments of the invention adapted for use where the malleus and the stapes footplate are present, but the stapes superstructure are partly or completely absent, the ossicular prosthesis includes the connecting element and the linking element. The connecting element is preferably configured to touch the malleus at one end and a fulcrum at the other end. The prosthesis touches the stapes footplate via the linking element, which is connected to the connecting element or touches the connecting element.

The linking element may be embodied integrally with the connecting element, or it may be produced separately from the connecting element. The linking element is preferably embodied as rod-shaped for better contact with the stapes footplate. The linking element touches or is connected to the connecting element preferably at between ends of the connecting element particularly at about half of the length of the connecting element.

The fulcrum is a structure in a middle ear that the connecting element is able to turn on when implanted. The fulcrum is not a part of the prosthesis. The fulcrum is preferably selected on the posterior wall of the middle ear particularly on aditus to mastoid antrum or on facial recess. It is particularly preferred that the fulcrum is selected near fossa incudis. However, it can be on elsewhere, for instance, on a reconstructed part of the middle ear particularly reconstructed posterior wall of the middle ear or on another implanted structure.

Additional aims and advantages of the invention will become apparent to those skilled in the art upon reference to the detailed description taken in conjunction with the provided figures.

### Brief description of drawings

Prior art FIG. 1 illustrates a prior art prosthesis 10, shown implanted between the tympanic membrane and the stapes head.

Prior art FIG. 2 illustrates a prior art prosthesis 20, shown implanted between the malleus and the stapes head.

Prior art FIG. 3 illustrates a prior art prosthesis 30, shown connected the malleus and the tympanic membrane to the stapes head.

FIG. 4 is a perspective view of a first embodiment of an ossicular prosthesis.

FIG. 5 illustrates implantation of the first embodiment of the ossicular prosthesis connecting the malleus to the stapes head.

FIG. 6 is a perspective view of a second embodiment of an ossicular prosthesis.

FIG. 7 illustrates implantation of the second embodiment of the ossicular prosthesis connecting the malleus to the stapes head.

FIG. 8 is a perspective view of a third embodiment of an ossicular prosthesis.

FIG. 9 is a perspective view of a fourth embodiment of an ossicular prosthesis.

FIG. 10 illustrates implantation of the fourth embodiment of the ossicular prosthesis connecting the malleus to the stapes footplate.

FIG. 11 is a perspective view of a fifth embodiment of an ossicular prosthesis.

FIG. 12 is an assembled view of the fifth embodiment of the ossicular prosthesis.

FIG.13 is a perspective view of a sixth embodiment of an ossicular prosthesis.

FIG.14 is an assembled view of the sixth embodiment of the ossicular prosthesis.

FIG.15 illustrates implantation of the sixth embodiment of the ossicular prosthesis connecting the malleus to the stapes footplate.

### Description of embodiments

Turning now to FIGS. 4, a first embodiment of an ossicular prosthesis 40 is shown. The first embodiment of the prosthesis 40 consists of a connecting element 42 having two ends 46, 46', and the connecting element 42 is configured to touch the malleus at the end 46, a fulcrum at the other end

46', and the stapes head at between its two ends 46, 46'. The parts of the ossicular prosthesis 40 that touch the malleus, the fulcrum, and the stapes head or whole of the prosthesis 40 are preferably made up of a material which is ductile and has no tendency to fix the structures it touches. One example of this type of material is titanium.

The end 46' may be embodied as having a convex surface or at least one protrusion to facilitate the turning of the connecting element 42 on the fulcrum. The end 46 may be embodied as having concave surface for better contact with the malleus.

In implanted position, the malleus and the fulcrum touch the ossicular prosthesis 40 at reverse side to which the stapes head touches the ossicular prosthesis 40 so that the stability of the ossicular prosthesis 40 is acquired.

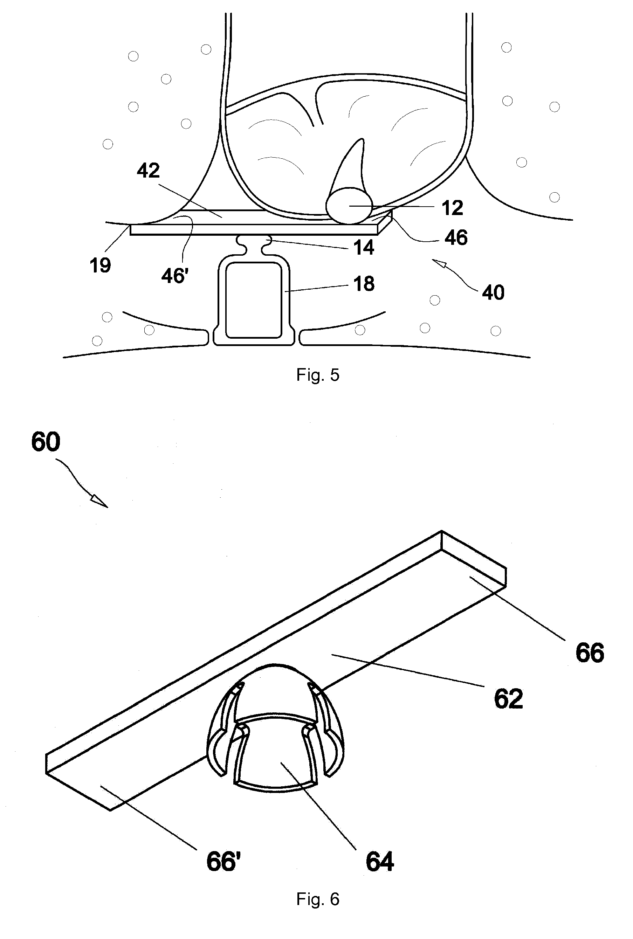

Referring to FIG. 5, the prosthesis 40 is used to reconstruct the ossicular chain between the malleus 12 and the head 14 of the stapes 18, where part or whole of the incus is absent. The end 46 of the connecting element 42 touches the malleus 12. The other end 46' of the connecting element 42 touches a fulcrum 19 on the posterior wall of the middle ear.

The connecting element 42 touches the stapes head 14 at about half of the length of the connecting element 42. The prosthesis 40 may be plastically deformed (or bent) to fit better to the anatomy.

In implanted position, the prosthesis 40 is very stable because the malleus 12 and the fulcrum 19 apply the connecting element 42 a force which is opposite in direction to the force applied to the connecting element 42 by the stapes head 14.

Turning to FIG. 6, a second embodiment of an ossicular prosthesis 60 is shown. The prosthesis 60 includes a connecting element 62 having two ends 66, 66' and a linking element 64. The linking element 64 is configured to connect the connecting element 62 to the stapes head. The linking element 64 is preferably embodied as bell-shaped for better contact with the stapes head. The connecting element 62 is configured to touch the malleus at the end 66 and a fulcrum at the other end 66'.

The linking element 64 may be integral with the connecting element 62 and is preferably situated at between two ends 66, 66' of the connecting element 62, preferably at about half of the length of the connecting element 62.

The linking element 64 may be separate from the connecting element 62 and touches or is connected to the connecting element 62 at between two ends 66, 66' of the connecting element 62, preferably at about half of the length of the connecting element 62.

If the linking element 64 only touches the connecting element 62 in implanted position; in other words, if the linking element 64 is not permanently fixed to the connecting element 62, the connecting element 62 may be embodied as having one or more concavities that is configured to provide a contact area for the linking element 64 and to prevent the linking element 64 from moving out of contact with the connecting element 62. Additionally, the linking element 64 may be embodied as having a convex surface or a protrusion for better contact with one of the concavities of the connecting element 62.

The parts of the ossicular prosthesis 60 that touch the malleus, the fulcrum, and the stapes head or whole of the ossicular prosthesis 60 are preferably made up of a material which is ductile and has no tendency to fix the structures it touches.

The end 66' may be embodied as having a convex surface or at least one protrusion to facilitate the turning of the connecting element 62 on the fulcrum. The end 66 may be embodied as having a concave surface for better contact with the malleus.

In implanted position, the malleus and the fulcrum touch the connecting element 62 at reverse side to which the linking element 64 touches or is connected to the connecting element 62 so that the stability of ossicular prosthesis 60 is acquired.

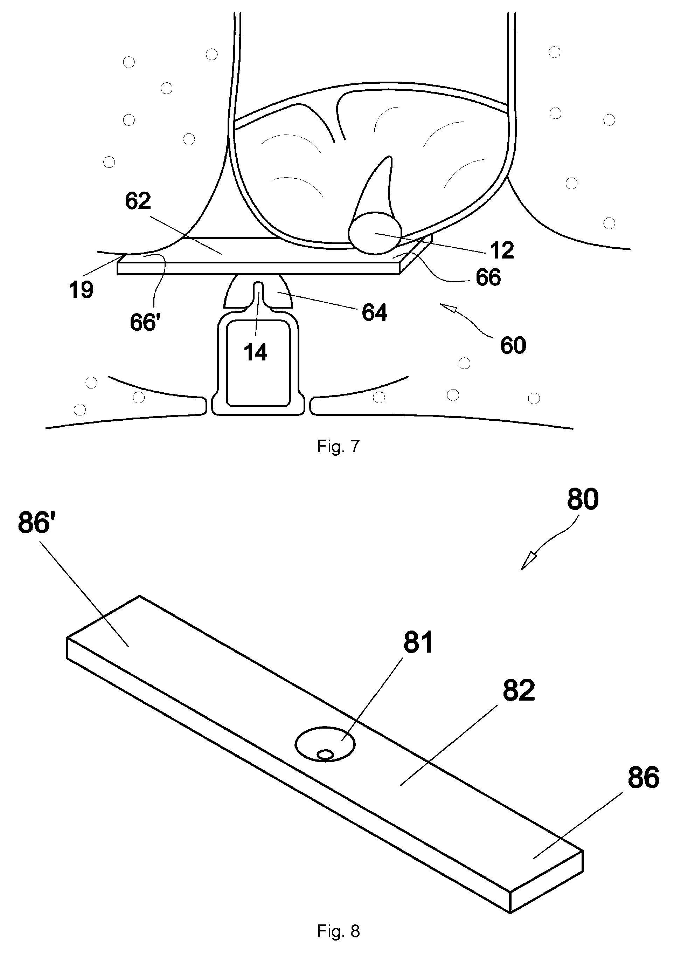

Referring to FIG. 7, the prosthesis 60 is used to reconstruct the ossicular chain between the malleus 12 and the stapes head 14, where part or whole of the incus is absent. The end 66 of the ossicular prosthesis 60 touches the malleus 12. The other end 66' of the prosthesis 60 touches the fulcrum 19 on the posterior wall of the middle ear. The linking element 64 is placed over the stapes head 14.

The connecting element 62 and/or the linking element 64 may be plastically deformed (or bent) to fit better to the anatomy. In implanted position, the prosthesis 60 is very stable because the malleus 12 and the fulcrum 19 apply the connecting element 62 a force which is opposite in direction to the force applied to the linking element 64 by the stapes head 14.

Referring to FIG. 8, a third embodiment of an ossicular prosthesis 80 is shown. The prosthesis 80 includes a connecting element 82 having two ends 86, 86' and at least one notch 81 suitable for contact with the stapes head. The connecting element 82 is configured to touch the malleus at the end 86, a fulcrum at the other end 86', and the stapes head with the notch 81.

The notch 81 is situated at between two ends 86, 86' of the connecting element 82. The parts of the ossicular prosthesis 80 that touch the malleus, the fulcrum, and the stapes head or whole of the prosthesis 80 are preferably made up of a material which is ductile and has no tendency to fix the structures it touches.

The end 86' may be embodied as having a convex surface or at least one protrusion to facilitate the turning of the connecting element 82 on the fulcrum. The end 86 may be embodied as having a concave surface for better contact with the malleus.

In implanted position, the malleus and the fulcrum touch the connecting element 82 at reverse side to which the notch 81 is on so that the malleus and the fulcrum apply the connecting element 82 a force that is opposite in direction to the force applied by the stapes head to the connecting element 82.

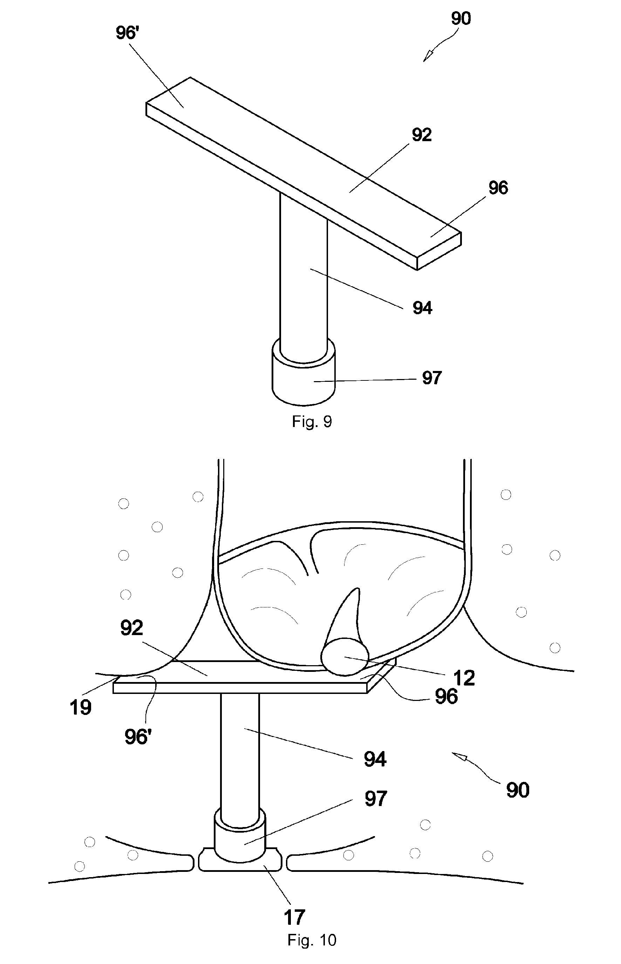

Turning now to FIG. 9, a fourth embodiment of an ossicular prosthesis 90 is shown. The prosthesis 90 includes a connecting element 92 having two ends 96, 96' and a linking element 94. The connecting element 92 is configured to touch the malleus at the end 96 and a fulcrum at the other end 96'. The linking element 94 is configured to connect the connecting element 92 to the stapes footplate. The linking element 94 is preferably embodied as rod-shaped.

The linking element 94 may be integral with the connecting element 92 and is situated at between two ends 96, 96' of the connecting element 92, preferably at about half of the length of the connecting element 92.

The linking element 94 may be separate from the connecting element 92 and touches or is connected to the connecting element 92 at between two ends 96, 96' of the connecting element 92, preferably at about half of the length of the connecting element 92.

If the linking element 94 touches the connecting element 92 in implanted position; in other words, if the linking element 94 is not permanently fixed to the connecting element 92, the connecting element 92 may be embodied as having one or more concavities that is configured to provide a contact area for the linking element 94 and to prevent the linking element 94 from moving out of contact with the connecting element 92. Additionally, the linking element 94 may be embodied as having a convex surface or a protrusion for better contact with one of the concavities of the connecting element 92.

Longitudinal axes of the connecting element 92 and the linking element 94 are preferably substantially perpendicular to each other in implanted position. Optionally, the linking element 94 may have an enlarged distal end 97 for better contact with the stapes footplate. The parts of the connecting element 92 that touch the malleus and the fulcrum or whole of the ossicular prosthesis 90 are preferably made up of a material which is ductile and has no tendency to fix the structures it touches.

The end 96' may be embodied as having a convex surface or at least one protrusion to facilitate the turning of the connecting element 92 on the fulcrum. The end 96 may be embodied as having a concave surface for better contact with the malleus.

The malleus and the fulcrum touch the connecting element 92 at reverse side to which the linking element 94 touches, or is connected to the connecting element 92.

Referring to FIG. 10, the prosthesis 90 is used to reconstruct the ossicular chain between the malleus 12 and a stapes footplate 17 where the stapes superstructure is missing (i.e., only the footplate 17 of the stapes remains). The end 96 of the connecting element 92 touches the malleus 12 and the other end 96' of the connecting element 92 touches the fulcrum 19 on the posterior wall of the middle ear. The linking element 94 touches or is connected to the connecting element 92. The enlarged distal end 97 of the linking element 94 is placed over the stapes footplate 17.

The connecting element 92 and/or the linking element 94 may be plastically deformed (or bent) to fit better to the anatomy. If the securing element 94 is not permanently fixed to the connecting element 92, the securing element 94 may be cut at the end that touches the connecting element 92 so that length of the prosthesis can be adjusted.

In implanted position, the prosthesis 90 is very stable because the malleus 12 and the fulcrum 19 apply the connecting element 92 a force that is opposite in direction to the force applied to the linking element 94 by the stapes footplate 17.

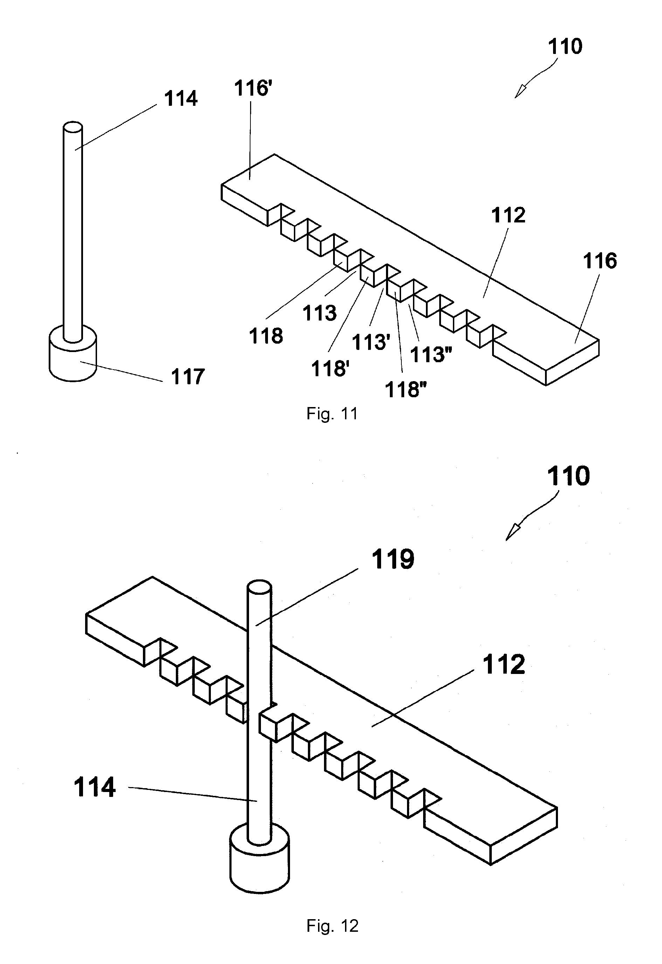

Turning to FIG. 11, a fifth embodiment of an ossicular prosthesis 110 is shown. The ossicular prosthesis 110 includes a connecting element 112 having two ends 116, 116' and a linking element 114 which is embodied as rod-shaped with a substantially circular cross section to facilitate the contact with the stapes footplate. The linking element 114 is configured to connect the connecting element 112 to the stapes footplate. The connecting element 112 is configured to touch the malleus at the end 116 and the fulcrum at the other end 116'.

In this embodiment, the connecting element 112 and the linking element 114 are separate parts. The connecting element 112 has one or more recesses 113, 113', 113" and processes 118, 118', 118". The diameter of the linking element 114 is chosen equal to or slightly bigger than the distance between adjacent processes 118, 118', 118" of the connecting element 112 so that the connecting element 112 and the linking element 114 can be coupled each other by placing the linking element 114, perpendicular to the connecting element 112, into one of the recesses 113, 113', 113" of the connecting element 112.

According to the anatomical differences, any one of the recesses 113, 113', 113" can be chosen for placement of the linking element 114 so that better fitting of the ossicular prosthesis 110 to different anatomical situations is acquired. Optionally, the linking element 114 may have a distal enlargement 117 for better contact with the stapes footplate.

The parts of the ossicular prosthesis 110 that touch the malleus, the fulcrum, and the stapes footplate or whole of the ossicular prosthesis 110 are preferably made up of a material which is ductile and has no tendency to fix the structures it touches.

The end 116' may be embodied as having a convex surface or at least one protrusion to facilitate the turning of the connecting element 112 on the fulcrum. The end 116 may be embodied as having a concave surface for better contact with the malleus.

Turning to FIG. 12, the prosthesis 110 is shown after the connecting element 112 and the linking element 114 coupled each other. After coupling, the part 119 of the linking element 114 past the connecting element 112 can be cut. By coupling the required length of the linking element 114 with the connecting element 112 and cutting the part 119 past the connecting element 112, the length of the prosthesis 110 can be adjusted.

In implanted position, the malleus and a fulcrum apply the connecting element 112 a force that is opposite in direction to the force applied to the connecting element 112 via the linking element 114 by the stapes footplate.

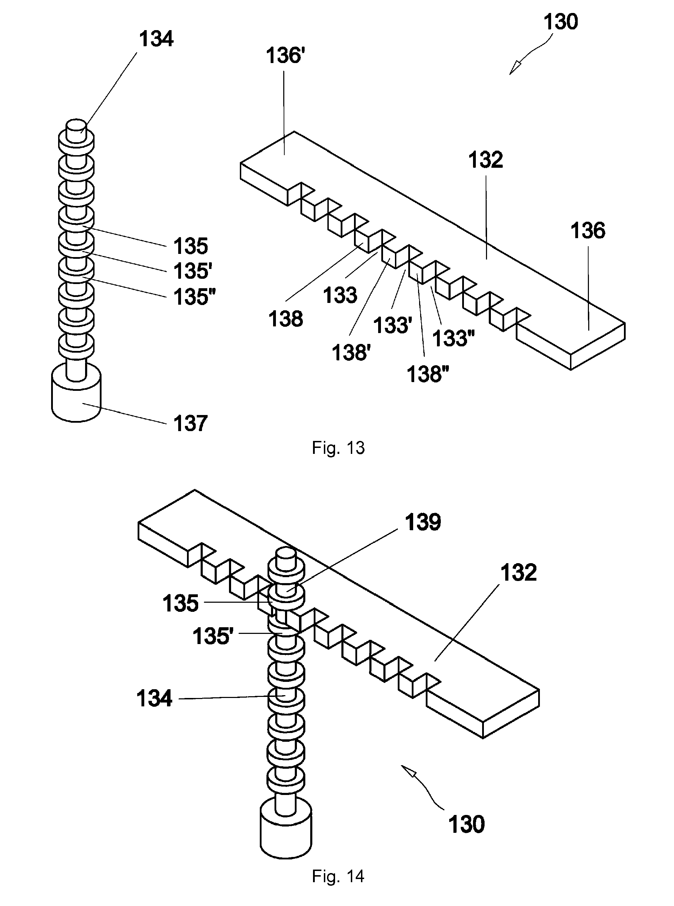

Referring to FIG. 13, a sixth embodiment of an ossicular prosthesis 130 is shown. The prosthesis 130 includes a connecting element 132 having two ends 136, 136' and a linking element 134 which is embodied as rod-shaped to facilitate the contact with the stapes footplate. The linking element 134 is configured to connect the connecting element 132 to the stapes footplate. The connecting element 132 is configured to touch the malleus at the end 136 and the fulcrum at the other end 136'.

In this preferred embodiment, the connecting element 132 and the linking element 134 are separate parts. The connecting element 132 has one or more recesses 133, 133', 133" and processes 138, 138', 138".

The linking element 134 is embodied as having one or more processes 135, 135', 135". The processes may be embodied as having an upper convex surface according to the implanted position. Optionally, the linking element has a distal enlargement 137 for better contact with the stapes footplate.

The processes 135, 135', 135" are configured in such a way that when the linking element 134 is placed, perpendicular to the connecting element 132, into one of the recesses 133, 133', 133" of the connecting element 132, the linking element 134 is prevented from moving perpendicularly to the connecting element 132.

In an alternative sixth embodiment of the ossicular prosthesis 130, the distance between adjacent processes 138, 138', 138" is made in such a way that when the linking element 134 is brought into one of the recesses 133, 133', 133" of the connecting element 132, only contact between the connecting element 132 and the linking element 134, in implanted position, will be the upper surface of only one of the processes 135, 135', 135" that not passed the connecting element 132.

The parts of the ossicular prosthesis 130 that touch the malleus and the fulcrum or whole of the ossicular prosthesis 130 are preferably made up of a material which is ductile and has no tendency to fix the structures it touches.

The end 136' may be embodied as having a convex surface or at least one protrusion to facilitate the turning of the connecting element 132 on the fulcrum. The end 136 may be embodied as having concave surface for better contact with the malleus.

Turning to FIG.14, the prosthesis 130 is shown after the connecting element 132 and the linking element 134 coupled each other. After coupling, the part 139 of the linking element 134 past the connecting element 132 can be cut.

The part 139 of the linking element 134 past the connecting element 132 may be cut in such a way that one of the processes 135, 135', 135" of the linking element 134 past the connecting element 132 (the process 135 in this illustrative figure) remains and touches the connecting element 132.

Alternatively, the part 139 of the linking element 134 past the connecting element 132 may be cut in such a way that none of the processes 135, 135', 135" of the linking element 134 on the part 139 remains and touches the connecting element 132 so that only part of the linking element 134 that touches the connecting element 132 will be one of the processes 135, 135', 135" of the linking element 134 that not passed the connecting element 132 (the process 135" in this illustrative figure).

By coupling the required length of the linking element 134 with the connecting element 132 and cutting the part 139 past the connecting element 132, the length of the prosthesis 130 can be adjusted. In implanted position, the malleus and the fulcrum apply the connecting element 132 a force which is opposite in direction to the force applied to connecting element 132 via the linking element 134 by the stapes footplate.

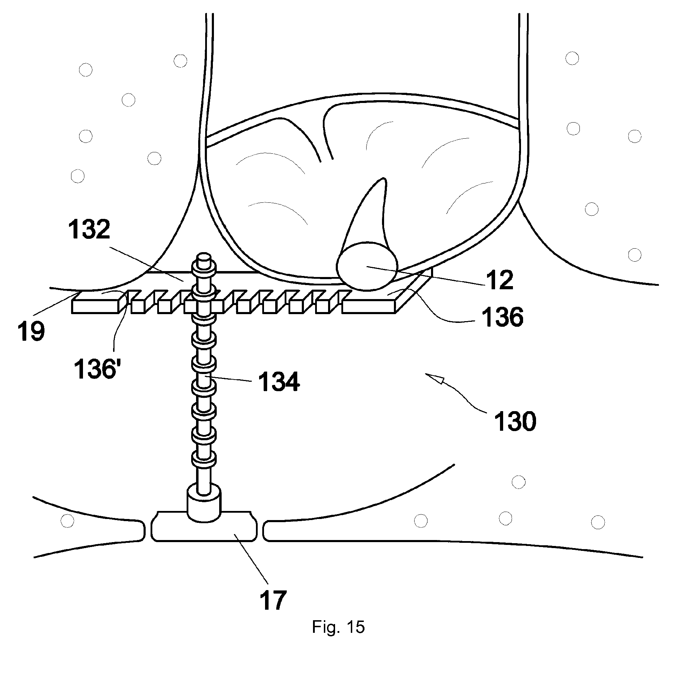

Referring to FIG. 15, the prosthesis 130 is used to reconstruct the ossicular chain between the malleus 12 and a stapes footplate 17 where the stapes superstructure is missing (i.e., only the footplate 17 of the stapes remains). The end 136 of the connecting element 132 touches the malleus 12 and the other end 136' of the connecting element 132 touches the fulcrum 19 on the posterior wall of the middle ear. The linking element 134 is coupled with the connecting element 132 and placed over the stapes footplate 17. The connecting element 132 and/or the linking element 134 may be plastically deformed (or bent) to fit better the anatomy.

In implanted position, the prosthesis 130 is very stable because the malleus 12 and the fulcrum 19 apply the connecting element 132 a force which is opposite in direction to the force applied to the linking element 134 by the stapes footplate 17.

There have been described and illustrated here in several embodiments of an ossicular prosthesis. While particular embodiments of the invention have been described, it is not intended that the invention be limited there to, as it is intended that the invention be as broad in scope as the art will allow and that the specification be read likewise. Thus, while particular structural materials and shapes have been disclosed, it will be appreciated that other structural materials and shapes can be used as well. Further, features and particulars of the various embodiments can be used interchangeably with the other embodiments described. It will therefore be appreciated by those skilled in the art that yet other modifications could be made to the provided invention without deviating from its spirit and scope as claimed.

## Claims

1. An ossicular prosthesis for implantation in a middle ear of a human, said ossicular prosthesis comprising a connecting element, **characterized in that** said connecting element is configured to be able to turn on a fulcrum in implanted position according to the vibration applied to said connecting element, the turning causing said connecting element to vibrate a structure that said connecting element is in physical contact with.

2. An ossicular prosthesis according to claim 1, wherein said fulcrum is selected from the group consisting of posterior wall of the middle ear, the aditus to mastoid antrum, facial recess, a reconstructed wall of an ear and another implanted structure.

3. An ossicular prosthesis according to claim 1 or 2, wherein said connecting element is configured to touch the malleus so that the vibration of the tympanic membrane cause said connecting element to vibrate the structure that said connecting element is in physical contact with.

4. An ossicular prosthesis according to claim 1 or 2, wherein said connecting element is configured to touch the malleus at one end and the fulcrum at the other end.

5. An ossicular prosthesis according to any one of claims 1 to 4, wherein said connecting element touches the stapes head at between and spaced apart from two ends of said connecting element.

6. An ossicular prosthesis according to any one of claims 1 to 4, further including a linking element which is integral with or separate from said connecting element, said linking element being configured to connect said connecting element to the stapes head or footplate so that the turning of said connecting element vibrates the stapes head or footplate via said linking element.

7. An ossicular prosthesis according to claim 6, wherein said linking element touches or is connected to said connecting element at between ends of said connecting element, preferably at about the half of the length of said connecting element.

8. An ossicular prosthesis according to claim 6 or 7, wherein said connecting element has one or more concavities configured to provide a contact area for said linking element and to prevent said linking element from moving out of contact with said connecting element.

9. An ossicular prosthesis according to claim 6, 7 or 8, wherein said linking element is embodied as bell-shaped for contact with the stapes head or rod-shaped for contact with the stapes footplate.

10. An ossicular prosthesis according to any one of claims 6 to 9, wherein said connecting element has one or more recesses for coupling said linking element when said linking element is placed into one of said recesses.

11. An ossicular prosthesis according to claim 10, wherein said linking element has one or more processes for preventing said linking element from moving perpendicularly to said connecting element when said linking element is placed into one of said recesses.

12. An ossicular prosthesis according to any one of claims 6 to 9, wherein said connecting element has one or more recesses having enough size for linking element to be placed into, and said linking element has one or more processes having enough size not to pass through said recesses of said connecting element.

13. An ossicular prosthesis according to any preceding claim, wherein the part of said connecting element that touches the fulcrum is embodied as having a convex surface or at least one protrusion to facilitate the turning of said connecting element on a fulcrum.

14. A linking element for use with an ossicular prosthesis according to claim 1, 2 or 3, wherein said linking element is configured to connect the connecting element to the stapes head or footplate.

15. A linking element according to claim 14, wherein said linking element have plurality of processes, said processes are designed to couple said connecting element when said connecting element is brought into adjacent two of said processes.
